# EUROPEAN PATENT APPLICATION

(11) **EP 3 130 661 A1**
(43) Date of publication of application: **15.02.2017**
(21) Application number: 15777368.0
(22) Date of filing: 06.04.2015
(51) Int. Cl.: C12N 1/00, C12M 1/00

(54) **CULTURE LIQUID FOR ANIMAL CELLS, AND CULTURE VESSEL**

(30) Priority: 07.04.2014 JP 2014078583
(71) Applicant: Toyo Seikan Group Holdings, Ltd., Shinagawa-ku Tokyo 141-8627 (JP)
(72) Inventor: OTA, Kyohei, Yokohama-shi Kanagawa 240-0062 (JP); TANAKA, Satoshi, Yokohama-shi Kanagawa 240-0062 (JP)
(74) Representative: Liebetanz, Michael
(86) International application number: PCT/JP2015/001920
(87) International publication number: WO 2015/155975

(57) **Abstract**

Provided is a culture medium for animal cells that can allow the performance of a highly gas-permeable culture container to be fully exhibited, whereby a high cell proliferation efficiency can be obtained. The culture medium for animal cells for enhancing the metabolism activity of a TCA cycle (tricarboxylic acid cycle) in respiration of animal cells, wherein the culture medium comprises, as an effective component, an amino acid metabolized to succinyl CoA that is an element constituting a TCA cycle at a concentration of 2.3 mmol/L to 6.0 mmol/L.

## Description

### Technical Field

The present invention relates to a technology of culturing cells, in particular, the present invention relates to a cell culture medium for animal cells and a culture container for improving proliferation efficiency of animal cells.

### Background Art

In recent years, in the field of production of pharmaceuticals, gene therapy, regenerative medical treatment, immunotherapy or the like, it is required to culture a large amount of cells, tissues, microorganisms or the like in an artificial environment efficiently.

Under such circumstances, cells and a culture medium are sealed in a culture container made of a gas-permeable film, and the cells are cultured in a large amount in a closed system.

In order to obtain excellent cell proliferation efficiency in such cell culture, the applicant of the present application developed a multilayer film having a high gas permeability and has already completed a highly gas-permeable culture container using the same (see Patent Document 1). It is considered that gas exchange required for cellular respiration can be conducted efficiently if cell culture is conducted by using such highly gas-permeable culture container, and cell culture efficiency was expected to be improved greatly by using such cell culture container.

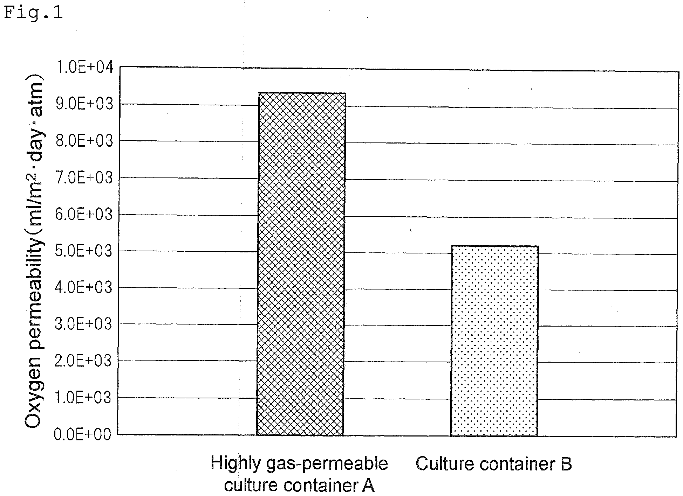

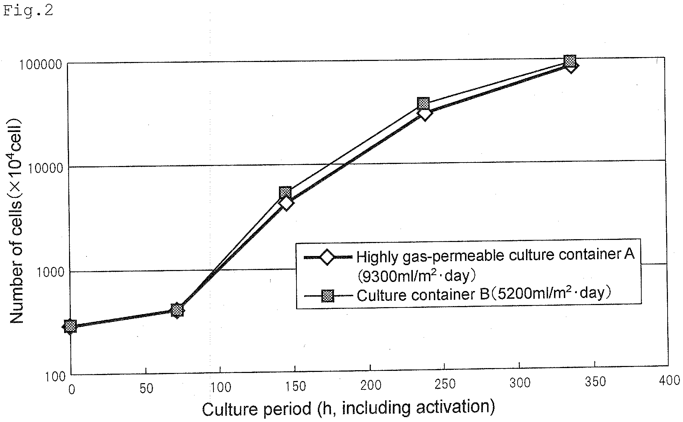

Under such circumstances, the applicant of the present application conducted cell culture for proliferating lymphocytes of a human being by using a culture container A, that is a highly gas-permeable culture container (oxygen permeability: 9300 ml/m²·day·atm) and a normal culture container B made of a conventional gas-permeable film (oxygen permeability: 5200 ml/m²·day·atm) as shown in Fig.1 (corresponding to Comparative Examples 1 and 2, given later). As a result, as shown in Fig. 2, data was obtained showing that almost no difference was found in cell proliferation efficiency between the culture containerA and the culture container B.

The reason therefor was not clearly elucidated. However, it was assumed that aerobic respiration enough to allow the performance of a highly gas-permeable culture container to be fully exhibited in cell culture was not conducted. Further, as the reason that sufficient aerobic respiration was not conducted, it was considered that the culture medium used for culture did not have sufficient nutrients required for aerobic respiration.

Here, cells utilize energy generated by respiration when they proliferate. The respiration is divided into glycolysis respiration and oxidative phosphorylation respiration, and ATP (adenosine triphosphate) is produced by these respirations.

The glycolysis respiration is anaerobic metabolism, and during the process of converting glucose into pyruvic acid, 2ATPs are produced for each glucose molecule, and lactic acid is generated from pyruvic acid. When lactic acid is generated, the pH of the culture medium is lowered, and proliferation efficiency is deteriorated.

The oxidative phosphorylation respiration is aerobic (using oxygen) metabolism, and converts pyruvic acid into acetyl CoA and supplies it to a TCA (tricarboxylic acid) cycle. Lactic acid is not generated, and NADH (nicotinamide adenine dinucleotide) or the like generated in the TCA cycle is supplied to an electron transport chain, and 38ATPs are produced for each glucose molecule. As mentioned above, in the aerobic circumstances, energy production efficiency is improved by the oxidative phosphorylation, and the amount of glucose consumed is reduced.

However, some of cells that conduct aerobic respiration do not use oxygen on purpose. For example, cancer cells do not undergo oxidative phosphorylation in aerobic circumstance, and generate ATP by glycolysis respiration. The reason therefor is thought to be as follows: since cancer cells undergo cell division actively, rather than oxidative phosphorylation that is advantageous for energy production, glycolysis (including a pentose phosphate pathway) that is advantageous for the production of nucleic acid or NADH (nicotinamide adenine dinucleotide phosphate) is used on purpose.

It was supposed that glycolysis was used actively and oxidative phosphorylation that can fully utilize the performance of a highly gas-permeable culture container was not conducted since a large amount of lactic acid is produced in the culture of lymphocytes mentioned above.

However, it was not clear whether proliferation efficiency of cells was improved as expected if oxidative phosphorylation that can utilize sufficiently the performance of a highly gas-permeable culture container is conducted. In addition, it was also unclear how the ratio of oxidative phosphorylation in cellular respiration could be increased.

As for related art documents in which statements are made on increasing the ratio of oxidative phosphorylation in cellular respiration, Patent Document 2 and Patent Document 3 can be given.

Patent Document 2 discloses a method for reducing glucose consumption and formation of lactate when cells of mammals are proliferated. Specifically, a method is disclosed in which, in the presence of citric acid or citrate in an amount of 1 to 50 mmol/L, proteins are produced by culturing cells of mammals in a culture medium that does not contain a serum. According to this method, since glucose consumption and formation of lactate are reduced at the time of proliferating cells, the ratio of oxidative phosphorylation in cellular respiration is thought to be increased.

Patent Document 3 discloses a serum-free culture medium for improving cell proliferation by adding only limited types of amino acids; specifically, it discloses a serum-free culture medium obtained by adding arginine, aspartic acid, glutamic acid, isoleucine, leucine, lysine, serine, threonine and valine.

### Related Art Documents

### Patent Documents

Patent Document 1: Patent No. 5191970
Patent Document 2: Patent No. 5344094
Patent Document 3: JP-A-2004-275047

### Disclosure of the Invention

### Problems to be Solved by the Invention

However, by the technologies disclosed in these patent documents, it was difficult to improve cell proliferation efficiency largely by allowing the performance of a highly gas-permeable culture container to be exhibited sufficiently.

As a result of extensive studies, the inventors of the present invention have found that, by incorporating, as effective components, into a culture medium amino acids that are metabolized to succinyl CoA as an element constituting a TCA cycle at a concentration of 2.3 mmol/L to 6.0 mmol/L, the metabolism activity of the TCA cycle in cellular respiration is improved, whereby the ratio of oxidative phosphorylation can be increased. In addition, when cell culture is conducted by using a highly gas-permeable culture container in which this culture medium is filled, as compared with a case where a normal gas-permeable culture container is used, cell proliferation efficiency could be successfully improved. The present invention has been completed based on this finding.

That is, the present invention is aimed at providing an animal cell culture medium and a culture container that can allow the performance of a highly gas-permeable culture container to be fully exhibited and can attain a high cell proliferation efficiency.

### Means for Solving the Problem

In order to attain the above-mentioned object, the culture medium for animal cells according to the present invention is a culture medium for animal cells for enhancing the metabolism activity of a TCA cycle (tricarboxylic acid cycle) in respiration of animal cells, wherein the culture medium comprises, as essential components, amino acids metabolized to succinyl CoA that is an element constituting a TCA cycle at a concentration of 2.3 mmol/L to 6.0 mmol/L.

Further, the culture container according to the present invention has a configuration in which the culture medium for animal cells according to the present invention is filled in a gas-permeable container.

### Advantageous Effects of the Invention

According to the present invention, it is possible to provide a culture medium for animal cells and a culture container that can allow the performance of a highly gas-permeable culture container to be fully exhibited, and attain a high cell proliferation efficiency

### Brief Description of the Drawings

Fig. 1 is a graph showing oxygen permeability of each of a highly gas-permeable culture container and a normal gas-permeable culture container;
Fig. 2 is a view showing the results of cell culture conducted by filling a highly gas-permeable culture container and a normal gas-permeable culture container respectively with a conventional culture medium for animal cells;
Fig. 3 is a view for explaining a TCA cycle in cellular respiration;
Fig. 4 is a view showing the amino acid composition of the culture medium for animal cells according to the embodiment of the present invention and the amino acid composition of a conventional culture medium for animal cells;
Fig. 5 is a view of a line graph showing the results of comparison in number of cultured cells in cell culture conducted by using the highly gas-permeable culture container and the normal gas-permeable culture container in which the culture medium for animal cells according to the embodiment of the present invention and a conventional culture medium for animal cells are respectively filled;
Fig. 6 is a view of a bar graph showing the results of comparison in number of cultured cells in cell culture conducted by using the highly gas-permeable culture container and the normal gas-permeable culture container in which the culture medium for animal cells according to the embodiment of the present invention and a conventional culture medium for animal cells are respectively filled;
Fig. 7 is a view of a bar graph showing the results of comparison in glucose consumption ratio in cell culture conducted by using the highly gas-permeable culture container and the normal gas-permeable culture container in which the culture medium for animal cells according to the embodiment of the present invention and a conventional culture medium for animal cells are respectively filled;
Fig. 8 is a view of a bar graph showing the results of comparison in lactic acid production ratio in cell culture conducted by using the highly gas-permeable culture container and the normal gas-permeable culture container in which the culture medium for animal cells according to the embodiment of the present invention and a conventional culture medium for animal cells are respectively filled;
Fig. 9 is a view of a graph showing the amino acid composition of each of the culture medium for animal cells according to the embodiments of the present invention and the amino acid composition of conventional culture medium for animal cells;
Fig. 10 is a view of a bar graph showing the results of comparison in cell proliferation efficiency in cell culture conducted by using the highly gas-permeable culture container and the normal gas-permeable culture container in which the culture medium for animal cells according to the embodiment of the present invention and a conventional culture medium for animal cells are respectively filled;
Fig. 11 is a view showing oxygen permeability of two types of highly gas-permeable culture containers and a normal gas-permeable culture container;
Fig. 12 is a view of a bar graph showing the results of comparison in cell proliferation efficiency in cell culture conducted by using two types of highly gas-permeable culture containers and the normal gas-permeable culture container in which various culture medium for animal cells according to the embodiment of the present invention and a conventional culture medium for animal cells are respectively filled;
Fig. 13 is a view showing the amino acid composition of the culture medium for animal cells according to the embodiment of the present invention and a conventional culture medium for animal cells;
Fig. 14 is a view showing the composition of each of the culture medium for animal cells according to the embodiment of the present invention; and
Fig. 15 is a view of a bar graph showing the results of comparison in cell proliferation efficiency in cell culture conducted by using the highly gas-permeable culture container in which various culture medium for animal cells according to the embodiment of the present invention and a conventional culture medium for animal cells are filled.

### Mode for Carrying out the Invention

Hereinbelow, one embodiment of the culture medium for animal cells will be explained in detail.

The culture medium for animal cells according to the present embodiment is a culture medium for animal cells for enhancing the activity of metabolism of a TCA cycle in respiration of animal cells, and comprises, as effective components, essential amino acids at a concentration of 6.0 mmol/L to 15 mmol/L.

The essential amino acids in the present embodiment mean 12 types of amino acids including completely essential amino acids and semi-essential amino acids, and are cysteine, threonine, tryptophan, leucine, lysine, arginine, histidine, isoleucine, valine, methionine, tyrosine and phenylalanine. The cell culture medium for animal cells according to the present embodiment comprises all of these essential amino acids.

A larger essential amino acid content in a cell culture medium for animal cells does not always lead to improvement in cell proliferation efficiency. That is, when an amino acid is metabolized, ammonia is generated as a nitrogen-based waste. If ammonia is accumulated in the culture medium, the pH of the culture medium is increased to deteriorate the culture environment, whereby proliferation of cells is inhibited. Therefore, when preparing a culture medium for animal cells, it is better to suppress the total content of amino acids to be low.

In this respect, in order to improve cell proliferation efficiency preferably, it is more preferred that the content of the essential amino acids in the cell culture medium for animal cells according to the present embodiment be 6.5 mmol/L to 13 mmol/L, with 6.9 mmol/L to 11 mmol/L being further preferable.

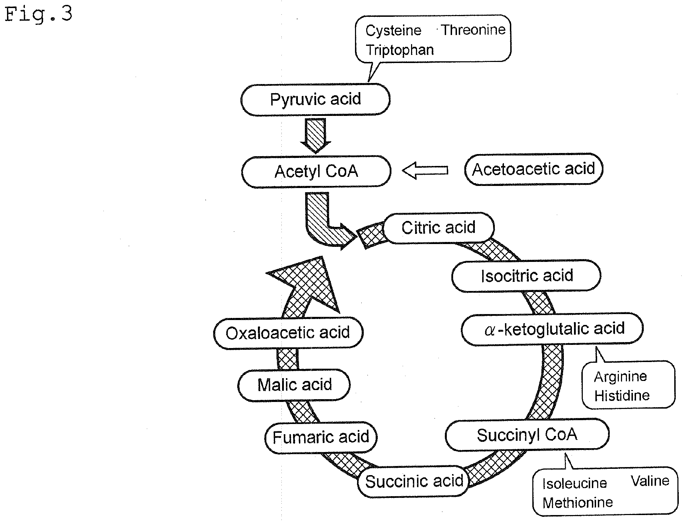

As shown in Fig. 3, the TCA cycle in cellular respiration has carboxylic acids such as succinyl CoA, α-ketoglutaric acid and pyruvic acid as the constituent molecules thereof.

By the culture medium for animal cells according to the present embodiment, by incorporating essential amino acids at the above-mentioned concentration, as compared with conventional culture medium for animal cells, it is possible to increase the supply amount of carbon sources metabolized to a carboxylic acid that is a constituent molecule of a TCA cycle, and to activate oxidative phosphorylation in cellular respiration.

As a result, the culture medium for animal cells according to the present embodiment can increase oxygen demand of cells, and can realize cell proliferation efficiency that suits to the performance of a gas-permeable culture container.

Regarding the essential amino acid to be contained in the cell culture for animal cells according to the present embodiment, it is preferred that amino acids to be metabolized to succinyl CoA as a constituent molecule of a TCA cycle be contained at a concentration of 2.3 mmol/L to 6.0 mmol/L, more preferably 2.3 mmol/L to 5.0 mmol/L, with 2.7 mmol/L to 4.0 mmol/L being further preferable.

By incorporating into the culture medium for animal cells according to the present embodiment amino acids metabolized to succinyl CoA at such a concentration range, it becomes possible to particularly improve the cell proliferation efficiency.

As the amino acids metabolized to succinyl CoA, it is preferable to use at least any of isoleucine, valine and methionine, and it is more preferable to use all of these.

In the essential amino acids to be contained in the culture medium for animal cells according to the present embodiment, by increasing the content of amino acids metabolized to α-ketoglutaric acid that is a constituent molecule of a TCA cycle, it is also possible to improve cell proliferation efficiency.

As the amino acid metabolized to α-ketoglutaric acid, it is preferable to use at least any of arginine and histidine, and it is more preferable to use all of them.

In the essential amino acids contained in the culture medium for animal cells according to the present embodiment, by increasing the content of amino acids metabolized to pyruvic acid that is a constituent molecule of a TCA cycle, it is also possible to improve cell proliferation efficiency.

As the amino acids metabolized to pyruvic acid, it is preferable to use at least any of cysteine, threonine and tryptophan, and it is more preferable to use all of them.

Here, the reasons for the fact that the cell proliferation efficiency can be particularly improved by incorporating into the culture medium for animal cells amino acids metabolized to succinyl CoA at the above-mentioned concentration range are not clearly elucidated. However, it can be considered that the reaction leading to the formation of succinyl CoA in a TCA cycle does not proceed smoothly is one of these reasons.

That is, since there are two enzymes, i.e. isocitrate dehydrogenase (converting isocitric acid to α-ketoglutaric acid) and oxaglutarate dehydrogenase (converting α-ketoglutaric acid to succinyl CoA), that regulate metabolism act immediately before forming succinyl CoA, the reaction leading to the formation of succinyl CoA does not proceed smoothly. By the culture medium for animal cells, since amino acids metabolized to succinyl CoA are supplied in a large amount, the reaction in a TCA cycle at the above-mentioned part where a reaction tends to be delayed can proceed smoothly, whereby cell proliferation efficiency can be particularly improved.

Further, the fact that amino acids metabolized to succinyl CoA are supplied as a carbon source of a substance to be synthesized through a TCA cycle is thought to be as one of these reasons. In particular, after metabolizing to succinyl CoA, these amino acids are used for synthesis of aminolevulinic acid. Aminolevulinic acid is converted to a hem through a porphyrin synthesis, and then used for synthesis of cytochrome c that is required for an electron transport chain. An electron transport chain is a metabolic pathway that consumes NADH generated in a TCA cycle to produce ATP Therefore, by reinforcing synthesis of cytochrome, energy is produced more rapidly. Further, an excessive amount of NADH inhibits a TCA cycle. Accordingly, it can be considered that, due to consumption of NADH by reinforcement of an electron transport chain, the entire TCA cycle can proceed smoothly.

In this respect, in the culture medium for animal cells according to the present embodiment, it is also preferred that cell proliferation efficiency be improved by incorporating other various nutrients that can supply succinyl CoA to a TCA cycle.

As such nutrients, an amino acid that supplies α-ketoglutaric acid mentioned above can be given, for example. In addition to arginine and histidine, glutamine, glutamic acid, proline or the like can be given.

In addition, as such nutrients, odd-chain fatty acids and lipids including the same (triglyceride, phospholipid), an ester or the like can be given. The odd-chain fatty acid means a fatty acid having an odd number of carbon chains, and propionic acid, pelargonic acid or the like can be given as specific examples thereof.

Animal cells to be cultured by using the culture medium for animal cells according to the present embodiment are not particularly restricted. However, induced pluripotent stem cells (iPS cells), embryonic stem cells (ES cells), mesenchymal stem cells, hematopoietic stem cells, neural stem cells, epithelial cells, muscle cells, cardiac muscle cells, osteoblasts, pancreatic islet beta (β) cells and the like can be given. By the culture medium for animal cells according to the present embodiment, lymphoid cells such as human-derived lymphocytes, hybridoma that produces monoclonal antibodies or the like can be preferably proliferated.

The cell culture conducted by using the culture medium for animal cells according to the present embodiment can be applied to any of semi-batch culture, batch culture and continuous culture.

The culture container according to the present embodiment is characterized in that the culture medium for animal cells according to the present embodiment is filled in a gas-permeable container.

In particular, by conducting cell culture by filling a highly gas-permeable culture container with the culture medium for animal cells according to the present embodiment, cell proliferation efficiency can be significantly improved.

The oxygen permeability of such a highly gas-permeable culture container is preferably 6000 ml/m²· day·atm or more, more preferably 7500 ml/m²·day·atm or more, and further preferably 9000 ml/m²·day·atm or more.

As mentioned above, by the culture medium for animal cells and the culture container according to the present embodiment, it is possible to increase oxygen demand of cells by activating a TCA cycle with essential amino acids at a prescribed concentration and to sufficiently utilize the performance of a highly gas-permeable culture container, and as a result, a high cell proliferation efficiency suited to its gas permeability can be obtained. In particular, when used by being filled in a highly gas-permeable culture container, its advantageous effects can be greatly exhibited.

In addition, by activating a TCA cycle, production of metabolic wastes (lactic acid) can be suppressed, and consumption of sugar (glucose) can also be suppressed.

Further, since production of metabolic wastes and consumption of sugar can be suppressed, the amount of a culture medium necessary for culture can be reduced.

### EXAMPLES

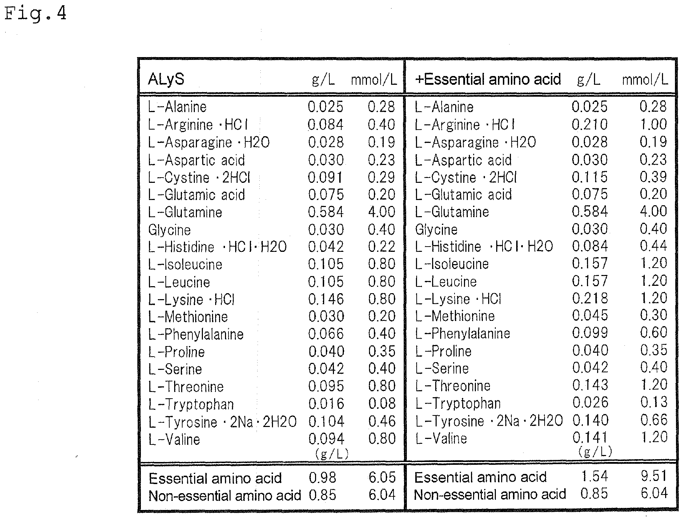

Hereinbelow, experiments conducted in order to confirm the advantageous effects of the culture medium for animal cells and the culture container according to the embodiment of the present invention will be explained with reference to Figs. 4 to 12.

### [Experiment 1]

Cell culture by using a highly gas-permeable culture container filled with the culture medium for animal cells according to the present embodiment (Example 1), cell culture by using a normal gas-permeable culture container filled with the culture medium for animal cells according to the present embodiment (Example 2), cell culture by using a highly gas-permeable culture container filled with a conventional culture medium (Comparative Example 1) and cell culture by using a normal gas-permeable culture container for animal cells filled with a conventional culture medium for animal cells (Comparative Example 2) were respectively conducted under the following conditions.

### (1) Culture medium for animal cells

First, as a conventional culture medium for animal cells, an ALyS-based medium (ALyS505N-7, manufactured by Cell Science & Technology Institute) was used. The amino acid composition thereof is shown in the column of "ALyS" in Fig. 4. The concentration of the essential amino acid in the ALyS-based medium was 6.05 mmol/L, and the concentration of an amino acid metabolized to succinyl CoA was 1.8 mmol/L.

Subsequently, as the culture medium for animal cells according to the present embodiment, one obtained by adding to an ALyS culture medium 12 types of essential amino acids at a prescribed concentration was used. The amino acid composition thereof is shown in the column of "+essential amino acid" in Fig. 4 (hereinbelow, the culture medium for animal cells in the present embodiment may be referred to as the "+ essential amino acid". The concentration of the essential amino acid in the +essential amino acid was 9.51 mmol/L and the concentration of an amino acid metabolized to succinyl CoA was 2.7 mmol/L.

### (2) Culture container

As the highly gas-permeable culture container, a highly gas-permeable culture container A shown in Fig. 1 (oxygen permeability: 9300 ml/m²·day·atm) was used. The highly gas-permeable culture container A was made of LLDPE (linear low-density polyethylene) and had a dimension of 200 mm × 200 mm.

Further, as the normal gas-permeable culture container, culture container B shown in Fig. 1 (oxygen permeability: 5200 ml/m²·day·atm) was used. The culture container B was made of EVA (ethylene-vinyl acetate copolymer resin) and had a dimension of 200 mm × 200 mm.

### (3) Experimental method

First, prior to the proliferation culture of cells, activation culture of cells was conducted. Specifically, anti-CD3 antibodies were solid-phased on a polystyrene-made dish. As the culture medium, one obtained by adding serum to an ALyS-based medium (ALyS505N-7+ serum 10%) was used. 296 × 10⁴ human peripheral blood mononuclear cells (hPBMC, manufactured by Cell Applications, Inc.) were inoculated to the dish together with the culture medium. Then, the resultant was cultured in an incubator at 37°C for 3 days at a CO₂ concentration of 5%.

Subsequently, proliferation culture of the activated cells was conducted. Specifically, for each Example and Comparative Example, activated cells and 500 ml of the culture medium for animal cells were poured into the above-mentioned culture container, and cultured for 11 days in an incubator at 37°C at a CO₂ concentration of 5%.

After 72.5 hours, 145.5 hours, 238.5 hours and 336.5 hours from the start of the culture including the activation, the number of cells was counted. The number of cells was counted by using a hemocytometer.

At the time of starting the culture and at the time of completing the culture (after 336. 5 hours), concentrations of glucose and lactic acid were measured. Concentration of glucose was measured by using iSTAT CG8+ (manufactured by Fuso Pharmaceutical Industries, Ltd.) and concentration of lactic acid was measured by using iSTAT CG4+ (manufactured by Fuso Pharmaceutical Industries, Ltd.). By calculating the consumed amount (mg) of glucose and the produced amount (mg) of lactic acid, glucose consumption ratio and lactic acid production ratio per unit cell proliferation (10⁸ cells) were calculated.

### (4) Experimental results

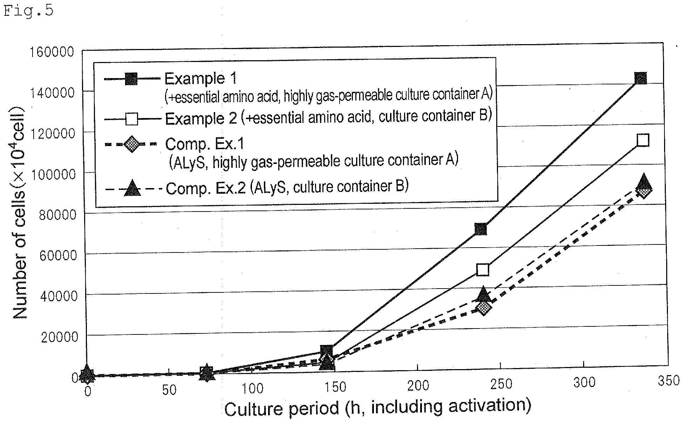

As shown in Fig. 5, when the culture medium for animal cells (+essential amino acid) according to the present embodiment was used, the number of cells cultured in the highly gas-permeable culture container (highly gas-permeable culture container A) (Example 1) was greatly increased as compared with the number of cells cultured in the normal gas-permeable culture container (culture container B) (Example 2).

That is, when the conventional culture medium for animal cells (ALyS) was used, almost no difference was found between the number of cells cultured in the highly gas-permeable culture container (Comparative Example 1) and the number of cells cultured in the normal gas-permeable culture container (Comparative Example 2). However, by using the culture medium for animal cells according to the present embodiment, the performance of the highly gas-permeable culture container could be fully exhibited, whereby proliferation efficiency of cells could be further improved.

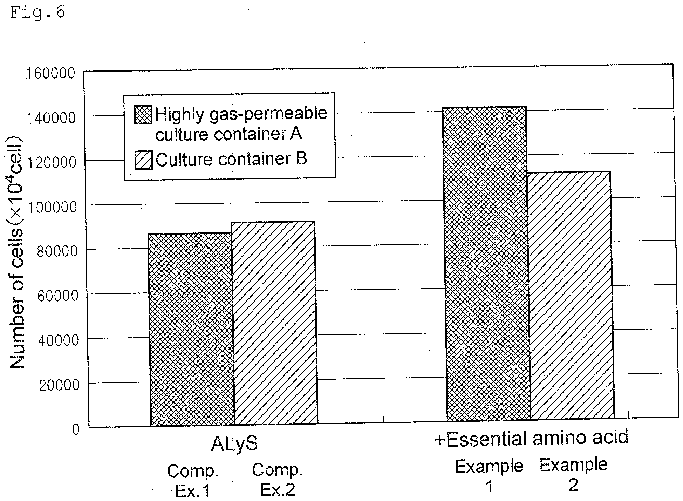

Fig. 6 is a bar graph showing the comparison results of the number of cultured cells at the time of completion of the culture period in each Example and each Comparative Example. When the culture medium for animal cells according to the present embodiment was used, the number of cells cultured in the highly gas-permeable culture container (Example 1: 141,600 × 10⁴) was increased by nearly 30% as compared with the number of cells cultured in the normal gas-permeable culture container (Example 2: 111,800 × 10⁴).

Further, the number of cells cultured in this normal gas-permeable culture container (Example 2) was increased by 20% or more as compared with the number of cells cultured in the normal gas-permeable culture container using the conventional culture medium for animal cells (Comparative Example 2: 91,300 × 10⁴).

That is, it was revealed that, by using the culture medium for animal cells according to the present embodiment, the cell proliferation efficiency by the normal gas-permeable culture container could be improved, and the cell proliferation efficiency by the highly gas-permeable culture container could be further improved.

The number of cells cultured in the highly gas-permeable culture container when the culture medium for animal cells according to the present embodiment was used (Example 1) was increased by 60% or more as compared with the number of cells cultured in the highly gas-permeable culture container filled with the conventional culture medium for animal cells (Comparative Example 1: 86,300 × 10⁴).

Therefore, it can be understood that, by the culture medium for animal cells according to the present embodiment, since the performance of the highly gas-permeable culture container can be fully exhibited, cell proliferation efficiency can be significantly increased.

Here, proliferation of cells is controlled by either the gas permeability of the culture container or the respiration activity of cells (oxygen utilization ability), whichever lower.

When the conventional culture medium for animal cells was used, the respiration activity of cells is lower than the gas permeability of the highly gas-permeable culture container and the normal gas permeable culture container, and hence almost no difference was seen between Comparative Example 1 and Comparative Example 2.

On the other hand, when the culture medium for animal cells according to the present embodiment was used, the respiration activity of cells was enhanced to be larger than the gas permeability, and it can be considered that cell proliferation efficiency was improved in Example 1 and Example 2.

Further, in the case of the normal gas-permeable culture container, when the conventional culture medium for animal cells is used, the low respiration activity was a factor for limiting the cell proliferation efficiency. By using the culture medium for animal cells according to the present embodiment, the respiration efficiency of cells was increased, and the gas permeability of the culture container became the factor for limiting the cell proliferation efficiency, and this difference contributes to improvement in cell proliferation efficiency from that in Comparative Example 2 to Example 2.

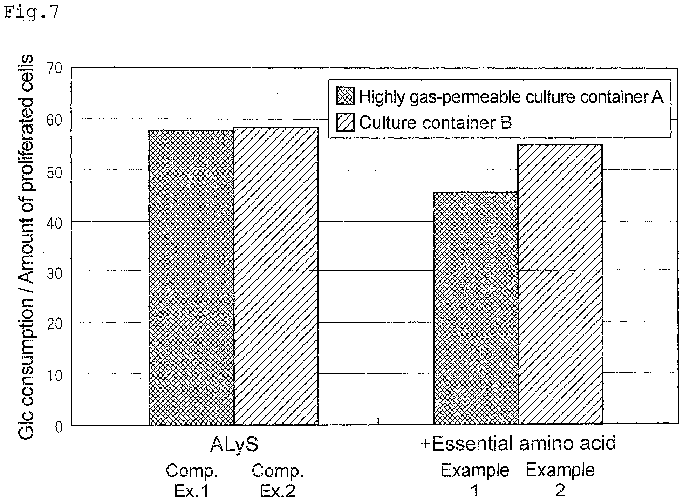

Fig. 7 is a bar graph showing the results of comparison in glucose consumption per amount of proliferated cells (Glc consumption (mg)/amount of proliferated cells (10⁸ cells), glucose consumption ratio) at the time of completion of cell culture period in each Example and each Comparative Example.

When the culture medium for animal cells (+essential amino acid) according to the present embodiment was used, the glucose consumption ratio by cell culture in the highly gas-permeable culture container (Example 1) was smaller by nearly 20% as compared with the glucose consumption ratio by cell culture in the normal gas-permeable culture container (Example 2). Further, the glucose consumption ratio in Example 1 was smaller by 20% or more as compared with the glucose consumption ratio in the highly gas-permeable culture container filled with the conventional culture medium for animal cells (ALyS) (Comparative Example 1).

That is, it can be understood that, since consumption of glucose is suppressed by using the culture medium for animal cells according to the present embodiment, the oxidative phosphorylation by cellular respiration is activated, whereby oxygen demand in cell culture is increased.

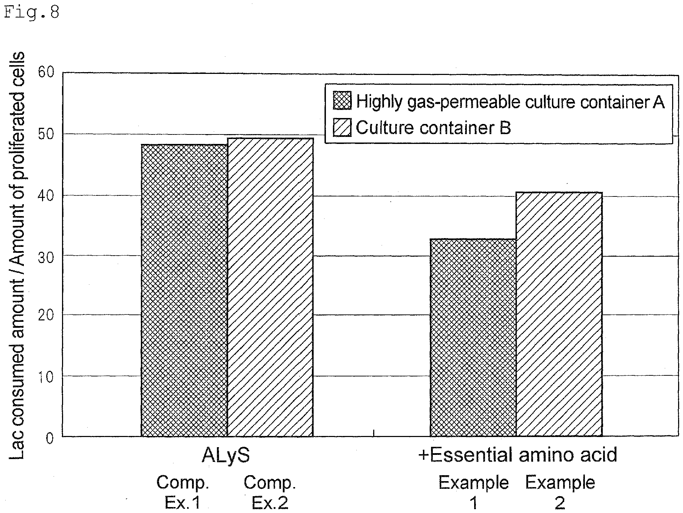

Fig. 8 is a bar graph showing the results of comparison in produced amount of lactic acid per amount of proliferated cells (Lac production amount (mg)/Amount of proliferated cells (10⁸ cells) lactic acid production ratio) at the time of completion of the culture period in each Example and each Comparative Example.

When the culture medium for animal cells (+essential amino acid) according to the present embodiment was used, the lactic acid production ratio by cell culture in the highly gas-permeable culture container (Example 1) was smaller by nearly 20% as compared with the lactic acid production ratio by cell culture in the normal gas-permeable culture container (Example 2). Further, the lactic acid production ratio of Example 1 was smaller than by 30% or more as compared with the lactic acid production ratio by cell culture in the highly gas-permeable culture container filled with the conventional culture medium for animal cells (ALyS).

That is, since production of lactic acid is suppressed by using the culture medium for animal cells according to the present embodiment, the oxidative phosphorylation by cellular respiration is activated, whereby oxygen demand in cell culture is increased.

### [Experiment 2]

In the essential amino acids contained in the culture medium for animal cells according to the present embodiment, an experiment for confirming the cell proliferation efficiency when the content of a prescribed amino acid metabolized to a constituent molecule of a TCA cycle is increased was conducted under the following conditions.

### (1) Culture medium for animal cells

As the conventional culture medium for animal cells, the same ALyS-based medium as that in Experiment 1 was used (Comparative Example 3).

As the culture medium for animal cells according to the present embodiment, one obtained by adding 12 types of essential amino acids to the ALyS-based medium as in Experiment 1 (+essential amino acid, Example 3), one obtained by adding essential amino acids (isoleucine, valine, methionine) metabolized to succinyl CoA to the ALyS-based medium (hereinafter often referred to as +succinyl CoA, Example 4), one obtained by adding essential amino acids metabolized to α-ketoglutaric acid (arginine and histidine) to the ALyS-based medium (hereinafter often referred to as +α-ketoglutaric acid, Example 5) and one obtained by adding essential amino acids metabolized to pyruvic acid (cysteine, threonine, tryptophan) to the ALyS-based medium (hereinafter often referred to as +pyruvic acid, Example 6) were used.

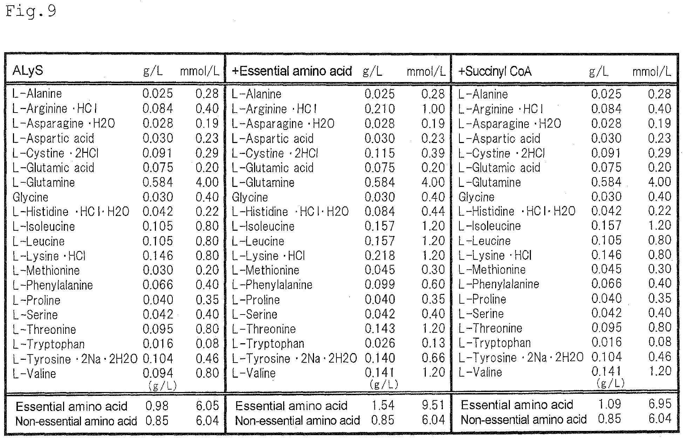

The amino acid composition of the culture medium for animal cells (+succinyl CoA) according to the present embodimentis shown in the column of "+succinyl CoA" in Fig. 9.

The +succinyl CoA was prepared by adding equivalent amounts of isoleucine, valine and methionine as those in +essential amino acid to the ALyS-based medium.

As for each of +α-ketoglutaric acid and +pyruvic acid, an amino acid was added to the ALyS-based medium in the same amount as that in +essential amino acid.

### (2) Culture container

As the highly gas-permeable culture container, the highly gas-permeable culture container A shown in Fig. 1 (oxygen permeability: 9300 ml/m²·day·atm) was used.

### (3) Experiment method

In the same manner as in Experiment 1, human peripheral blood mononuclear cells (hPBMC, manufactured by Cell Applications, Inc.) were activated, and the activated cells were subjected to proliferation culture. However, since these cells belong to a lot that is different from that in Experiment 1, respiration activity (enzyme utilization ability) of the cells is different from that in Experiment 1.

Then, the number of cells at the time of completion of the culture period was counted, and the proliferation ratio relative to the number of cells at the time of starting the culture period was calculated.

### (4) Experimental results

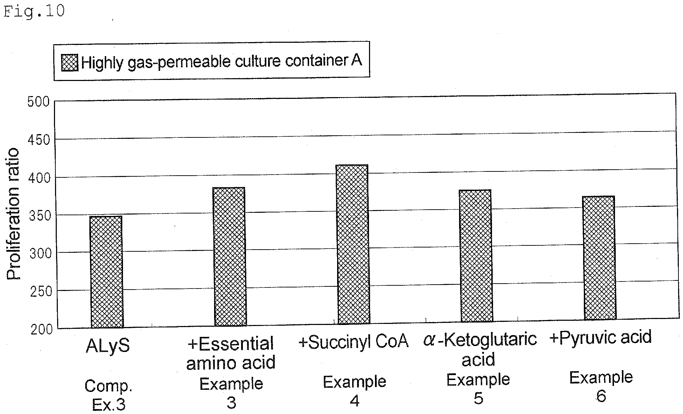

As shown in Fig. 10, the proliferation ratio in Comparative Example 3 was 346.5 times. Further, the proliferation ratio of +essential amino acid was 383.6 times (Example 3), the proliferation ratio of +succinyl CoA was 410.8 times (Example 4), the proliferation ratio of α-ketoglutaric acid was 375.1 times (Example 5) and the proliferation ratio of +pyruvic acid was 364.5 times (Example 6).

That is, the ratio of increase in cell proliferation ratio of the culture medium for animal cells according to the present embodiment relative to a case where the culture medium of Comparative Example 3 was used was 10.7% in the case of +essential amino acid (Example 3), 18.6% in the case of +succinyl CoA (Example 4), 8.3% in the case of α-ketoglutaric acid (Example 5) and 5.2.% in the case of +pyruvic acid (Example 6).

From the above, it was confirmed that the cell proliferation efficiency could be improved when, among the essential amino acids, only the prescribed amino acids were added to the conventional culture medium for animal cells.

In particular, the ratio of increase in cell proliferation ratio by +succinyl CoA obtained by adding isoleucine, valine and methionine was larger than 70% or more than that in the case of +essential amino acid.

Therefore, it was confirmed that the cell proliferation efficiency was significantly increased by the culture medium for animal cells (+succinyl CoA) according to the present embodiment.

### [Experiment 3]

An experiment for confirming the effect of improving the cell proliferation efficiency by the culture medium for animal cells (+succinyl CoA) according to the present embodiment was conducted under the following conditions.

### (1) Culture medium for animal cells

As the conventional culture medium for animal cells, the same ALyS-based medium to that in Experiment 1 was used (Comparative Examples 4 to 6).

As the culture medium for animal cells according to the present embodiment, one obtained by adding 12 types of essential amino acids to the ALyS-based medium as in Experiment 1 (+essential amino acid, Examples 7 to 9), one obtained by adding essential amino acids (isoleucine, valine, methionine) metabolized to succinyl CoA to the ALyS-based medium (+succinyl CoA, Examples 10 to 12), one obtained by adding essential amino acids metabolized to +succinyl CoA (isoleucine, valine, methionine) to the ALyS-based medium in an amount twice as large as that in Experiment 1 (hereinafter often referred to as +succinyl CoA × 2, Examples 13 to 15) were used.

### (2) Culture container

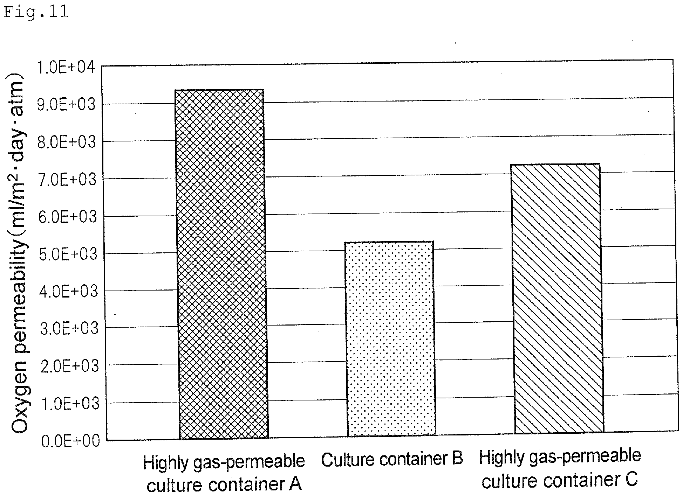

The culture container used in Experiment 3 is shown in Fig. 11.

The highly gas-permeable culture container (highly gas-permeable culture container A) is the same as that shown in Fig. 1 (oxygen permeability: 9300 ml/m²·day·atm), and the normal gas-permeable culture container (culture container B) is the same as that shown in Fig. 1 (oxygen permeability: 5200 ml/m²·day·atm).

Further, as the highly gas-permeable culture container (highly gas-permeable culture container C), one having an oxygen permeability of 7230 ml/m²·day·atm was used. The highly gas-permeable culture container C was made of LLDPE (linear low-density polyethylene) and had a dimension of 200 mm × 200 mm.

### (3) Experimental method

In the same manner as in Experiment 1, human peripheral blood mononuclear cells (hPBMC, manufactured by Cell Applications, Inc.) were activated, and the activated cells were subjected to proliferation culture. However, since these cells belong to a lot that is different from that in Experiment 1, the respiration activity (enzyme utilization ability) of the cells is different from that in Experiment 1. The culture period was 14 days (336 hours).

Then, the number of cells at the time of completion of the culture period was counted, and the proliferation ratio relative to the number of cells at the time of starting the culture period was calculated.

### (4) Experimental results

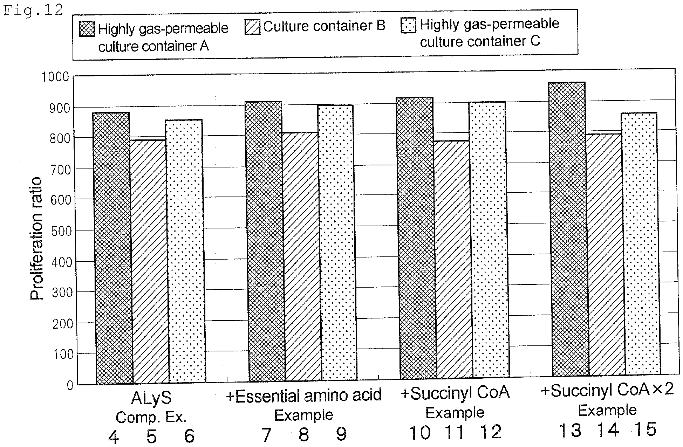

As shown in Fig. 12, when the conventional culture medium for animal cells was used, the cell proliferation ratios were 880.6 times in the highly gas-permeable culture container (highly gas-permeable culture container A) (Comparative Example 4), 790.7 times in the normal gas-permeable culture container (culture container B) (Comparative Example 5) and 853.3 times in the highly gas-permeable culture container (highly gas-permeable culture container C) (Comparative Example 6), respectively.

Further, when the culture medium for animal cells (+essential amino acid) according to the present embodiment was used, the cell proliferation ratios were 910.7 times in the highly gas-permeable culture container A (Example 7), 806.4 times in the culture container B (Example 8) and 893.7 times in the highly gas-permeable culture container C (Example 9).

Therefore, the ratio of increase in cell proliferation ratios of the culture medium for animal cells (+essential amino acid) according to the present embodiment relative to a case where the conventional culture medium for animal cells was used was 3.4% in the highly gas-permeable culture container A (Example 7), 2.0% in the culture container B (Example 8) and 4.7% in the highly gas-permeable culture container C (Example 9).

Further, when the culture medium for animal cells (+succinyl CoA) according to the present embodiment was used, the cell proliferation ratios were 917.3 times in the highly gas-permeable culture container A (Example 10), 773.7 times in the culture container B (Example 11) and 899.2 times in the highly gas-permeable culture container C (Example 12).

Therefore, the ratio of increase in cell proliferation ratio of the culture medium for animal cells (+succinyl CoA) according to the present embodiment relative to a case where the conventional culture medium for animal cells was used was 4.2% in the highly gas-permeable culture container A (Example 10), - 2.2% in the culture container B (Example 11) and 5.4% in the highly gas-permeable culture container C (Example 12).

Further, when the culture medium for animal cells (+succinyl CoA × 2) according to the present embodiment was used, the cell proliferation ratios were 957.7 times in the highly gas-permeable culture container A (Example 13), 788.5 times in the culture container B (Example 14) and 857.7 times in the highly gas-permeable culture container C) (Example 15).

Therefore, the ratio of increase in cell proliferation ratio of the culture medium for animal cells (+succinyl CoA × 2) according to the present embodiment relative to a case where the conventional culture medium for animal cells was used was 8.7% in the highly gas-permeable culture container A (Example 13), - 0.3% in the culture container B (Example 14) and 0.5% in the highly gas-permeable culture container C (Example 15).

Here, as mentioned above, proliferation of cells is controlled by either the gas permeability of the culture container or the respiration activity of cells (oxygen utilization ability), whichever lower, and is strongly affected by difference in respiration activity of individual cells. As for the cells used in Experiment 3, it is considered that the respiration activity thereof was larger than the gas permeability of the culture container B when the conventional culture medium for animal cells was used.

Therefore, it can be considered that, in the cell culture in the culture container B (Examples 8, 11 and 14), the gas permeability of the culture container B served as a factor for limiting the cell proliferation efficiency, and as a result, the respiration activity of cells could not be enhanced any longer even if the culture medium for animal cells according to the present embodiment was used.

On the other hand, in the highly gas-permeable culture container A and the highly gas-permeable culture container C, the gas permeability was still enough as compared with the respiration activity of cells, and hence, the respiration activity of the cells could be further enhanced, and the cell proliferation efficiency could be improved.

That is, in the cell culture by the highly gas-permeable culture container A (Examples 7, 10 and 13) and the cell culture by the highly gas-permeable culture container C (Examples 9, 12 and 15), since the gas permeability was larger than the respiration activity of cells, the difference between the gas permeability and the respiration activity of cells was exhibited as improvement in cell proliferation efficiency.

### [Experiment 4]

An experiment for confirming the effect of improving the cell proliferation efficiency by the culture medium for animal cells (+succinyl CoA) according to the present embodiment was conducted under the following conditions.

### (1) Culture medium for animal cells

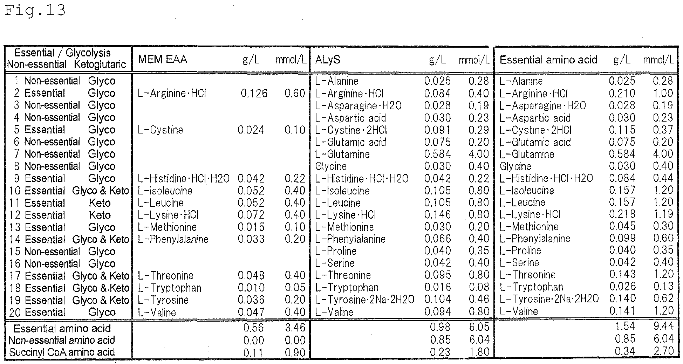

As the conventional culture medium for animal cells, the ALyS-based medium shown in Fig. 13 was used (concentration of an essential amino acid metabolized to succinyl CoA: 1.8 mmol/L, Comparative Example 7). This ALyS-based medium was the same as that used in Experiment 1.

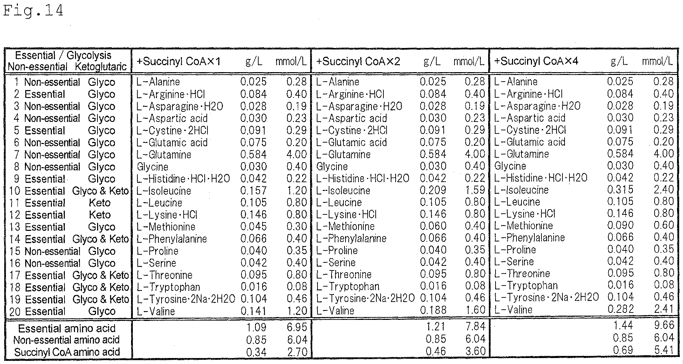

As the culture medium for animal cells according to the present embodiment, as shown in Fig. 14, one obtained by adding isoleucine, valine, methionine as essential amino acids metabolized to succinyl CoA to the ALyS-based medium as in Experiment 1 (hereinafter often referred to as +succinyl CoA × 1, concentration of essential amino acid metabolized to succinyl CoA, 2.7 mmol/L, Example 16), one obtained by adding isoleucine, valine, methionine as essential amino acids metabolized to succinyl CoA to the ALyS-based medium in an amount twice as large as the added amount in Experiment 16 (hereinafter often referred to as +succinyl CoA × 2, concentration of essential amino acid metabolized to succinyl CoA, 3.6 mmol/L, Example 17) and one obtained by adding isoleucine, valine and methionine as essential amino acids metabolized to succinyl CoA in an amount 4 times larger than the added amount in Experiment 16 (hereinafter referred to as +succinyl CoA × 4, concentration of essential amino acid metabolized to succinyl CoA was 5.4 mmol/L, Example 18) were used.

### (2) Culture container

For the culture container, as the highly gas-permeable culture container, the highly gas-permeable culture containerAshown in Fig. 1 (oxygen permeability: 9300 ml/m²·day·atm) was used.

### (3) Experimental method

In the same manner as in Experiment 1, human peripheral blood mononuclear cells (hPBMC, manufactured by Takara Bio Inc.) were activated, and the activated cells were subjected to proliferation culture. However, since these cells belong to a lot that is different from that in Experiment 1, the respiration activity (oxygen utilization ability) of the cells is different from that in Experiment 1. The culture period was 12 days (activation culture: 3 days, proliferation culture: 9 days, 284 hours in total).

Then, the number of cells at the time of completion of the culture period was counted, and the proliferation ratio relative to the number of cells at the time of starting the culture period was calculated.

### (4) Experimental results

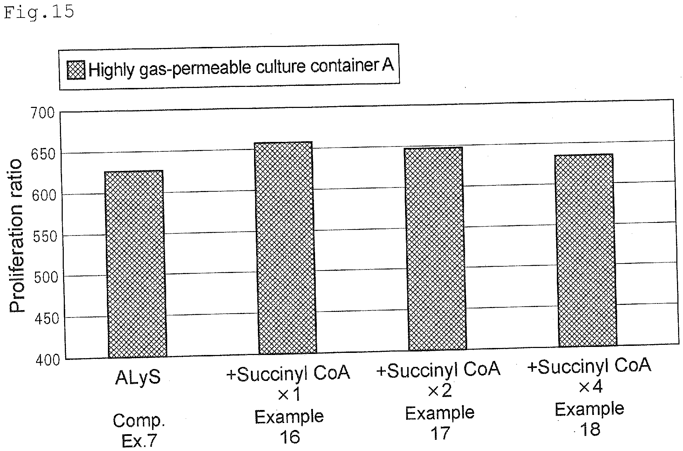

As shown in Fig. 15, the proliferation ratio in Comparative Example 7 was 626.7 times. The proliferation ratio of +succinyl CoA × 1 was 658.1 times (Example 16), the proliferation ratio of +succinyl CoA × 2 was 647.1 times (Example 17) and the proliferation ratio of +succinyl CoA × 4 was 634.8 times (Example 18).

That is, the ratio of increase in cell proliferation ratios of the culture medium for animal cells according to the present embodiment relative to a case where the culture medium of Comparative Example 7 was used was 5.0% in the case of +succinyl CoA (Example 16), 3.3% in the case of +succinyl CoA × 2 and 1.3% in the case of +succinyl CoA × 4 (Example 18).

From the above, it could be confirmed again that, by the culture medium for animal cells according to the present embodiment (+succinyl CoA), the cell proliferation efficiency can be improved.

The present invention is not limited to the above-mentioned embodiments or examples, and it is needless to say that various modifications are possible within the scope of the present invention.

For example, the following modifications can be made appropriately: the blending ratio of an amino acid metabolized to succinyl CoA in the culture medium for animal cells according to the present embodiment can be different from that in Examples, or other components metabolized to succinyl CoA can be added.

### Industrial Applicability

The present invention can preferably be used when a large amount of cells are produced by using a closed culture container in the field of production of pharmaceuticals, gene therapy, regenerative medical treatment, immunotherapy or the like.

## Claims

1. A culture medium for animal cells for enhancing the metabolism activity of a TCA cycle (tricarboxylic acid cycle) in respiration of animal cells, wherein the culture medium comprises, as effective components, amino acids metabolized to succinyl CoA that is an element constituting a TCA cycle at a concentration of 2.3 mmol/L to 6.0 mmol/L.

2. The culture medium for animal cells according to claim 1, wherein the amino acids metabolized to succinyl CoA are isoleucine, valine and methionine.

3. The culture medium for animal cells according to claim 1 or 2, wherein the animal cells are human-derived lymphocytes.

4. A culture container wherein the culture medium for animal cells according to any one of claims 1 to 3 is filled in a gas-permeable container.

5. The culture container according to claim 4, wherein the oxygen permeability of the gas-permeable container is 6000 ml/m²·day·atm or more.
